# EUROPEAN PATENT APPLICATION

(11) **EP 4 420 601 A1**
(43) Date of publication of application: **28.08.2024**
(21) Application number: 23158090.3
(22) Date of filing: 23.02.2023
(51) Int. Cl.: A61B 5/00, A61B 5/246, A61B 5/12

(54) **MAGNETIC RESONANCE GUIDED SELECTION OF SENSORY STIMULUS CONTROL COMMANDS FOR TINNITUS**

(71) Applicant: Koninklijke Philips N.V., 5656 AG Eindhoven (NL)
(72) Inventor: VAN EE, Raymond, Eindhoven (NL); MENA BENITO, Maria Estrella, Eindhoven (NL)
(74) Representative: Philips Intellectual Property & Standards

(57) **Abstract**

Disclosed herein is a medical system (100, 400) comprising a magnetic resonance imaging system (102) configured for acquiring k-space data (152, 156) of a brain of a subject (118) and a sensory stimulus system (122) configured for providing audio stimulation to the subject. Functional magnetic resonance imaging is used to select at least one subject specific sensory stimulus control command (166) from a predetermined set of sensory stimulus control commands 160 that maximize a change in neural activity in the at least one abnormal stimulus region of the audio cortex of the subject.

## Description

### TECHNICAL FIELD

The invention relates to functional magnetic resonance imaging, in particular to the use of functional magnetic resonance imaging for selecting audio stimulation for changing neural activity in subjects with tinnitus.

### BACKGROUND

Magnetic Resonance Imaging (MRI) may be used to measure detailed visualizations of the anatomical structure of a subject as well as directly measuring some biochemical reactions within the subject. For example, MRI can be used to measure the Hemodynamic response function within the brain to map neural activity. MRI techniques that measure neural activity within the brain are referred to herein as functional magnetic resonance imaging protocols for measuring brain activity.

International patent application publication WO 2008/062275 A1 discloses an apparatus and method for presenting high-quality auditory stimuli, receiving patient communication and providing noise cancellation within the environment of magnetic resonance imaging ("MRI") equipment. A microphone is positioned in a noise attenuated channel for recording the patient's voice. A microphone is disposed outside of a noise attenuated channel to directly record the sounds of MRI equipment during operation. The signals generated by the microphones are employed to reduce the output of noise generated by MRI equipment.

### SUMMARY

The invention provides for a medical system, a computer program, and a method in the independent claims. Embodiments are given in the dependent claims.

Embodiments may provide for an improved means of providing sensory stimulus, that includes audio stimulation, to a subject to modify neural activity in abnormal stimulus regions of the audio cortex. The neural response in the abnormal stimulus regions is measured using functional magnetic resonance imaging while different sensory stimulus commands are presented to the subject. Based on a change between a baseline functional magnetic resonance image (with not stimulus being presented) and a repetitively acquired reference functional magnetic resonance image (with a series of different sensory stimulus commands being used for each repetition) a set of subject specific sensory stimulus control commands can be selected from a predetermined set of sensory stimulus control commands.

For example, damage to hair cells in the cochlea of a subject may result in degraded hearing that is permanent. A difficulty is that portions of the auditory cortex may be abnormally stimulated or under stimulated due to reduced inhibition of neural connections between corresponding regions of the auditory cortex and portions of the cochlea with damaged hair cells. Embodiments may provide a means of determining which audio stimulation can be used to modify neural activity in these abnormal stimulus regions. This may for example provide for a means of providing for audio stimulation that assists in inhibiting regions of the auditory cortex that are stimulated because the damaged hair cells to which they are connected are no longer capable of providing inhibition.

In one aspect the invention provides for a medical system that comprises a magnetic resonance imaging system configured for acquiring k-space data of a brain of a subject. The medical system further comprises a sensory stimulus system that is configured for providing audio stimulation to the subject. The medical system further comprises a memory storing machine-executable instructions and pulse sequence commands. The pulse sequence commands are configured to control the magnetic resonance imaging system to acquire the k-space data according to a functional magnetic resonance imaging protocol for measuring brain activity in an audio cortex of the subject. The medical system further comprises a computational system that is configured for controlling the medical system.

Execution of the machine-executable instructions causes the computational system to receive a tonotopic mapping of an audio cortex mapping of the subject. The tonotopic mapping comprises a spatial mapping descriptive of audio frequency sensitivity response for the subject. Execution of the machine-executable instructions further causes the computational system to identify at least one abnormal stimulus region within the tonotopic mapping.

Execution of the machine-executable instructions further causes the computational system to acquire baseline k-space data by controlling the magnetic resonance imaging system with the pulse sequence commands. Execution of the machine-executable instructions further causes the computational system to reconstruct a baseline functional magnetic resonance image from the baseline k-space data.

Execution of the machine-executable instructions causes the computational system to repeatedly acquire reference k-space data by controlling the magnetic resonance imaging system with the pulse sequence commands while the sensory stimulus system is controlled with the one of a predetermined set of sensory stimulus control commands. Execution of the machine-executable instructions further causes the computational system to repeatedly control the sensory stimulus system with the one of a predetermined set of sensory stimulus control commands during acquisition of the reference k-space data. Each of the predetermined set of sensory stimulus control commands are configured to control the sensory stimulus system to present a predetermined audio stimulation to the subject. Execution of the machine-executable instructions further causes the computational system to reconstruct a reference functional magnetic resonance image from the reference k-space data. Execution of the machine-executable instructions further causes the computational system to repeatedly assign a numerical score to the one of the predetermined set of sensory stimulus control commands by detecting a change in neural activity in the abnormal stimulus region between the baseline functional magnetic resonance image and the reference functional magnetic resonance image, the score being representative of a decrease or increase in the neural activity in the abnormal stimulus regions.

After the predetermined set of sensory stimulus control commands have been presented to the subject using the sensory stimulus system and their effect on neural activity in the at least one abnormal stimulus region has been quantified with the numerical score, execution of the machine-executable instructions further causes the computational system to construct at least one subject-specific sensory stimulation control command by selecting sensory stimulus control commands from the predetermined set of sensory stimulus control commands that maximize a change in the neural activity in the at least one abnormal stimulus region using the numerical score for each representation of the predetermined set of sensory stimulus control commands.

In accordance with embodiments, the numerical score for each of the predetermined set of sensory stimulus control commands can be used to quantify how much of an effect they have on an increase or decrease in the neural activity in the at least one abnormal stimulus region. This may be beneficial because it may enable a means of producing the at least one subject-specific sensory stimulus control commands that have an effect on this region. This may have the effect that when this is repeatedly presented to the subject it may cause a reduction in the symptoms of tinnitus.

It is also noted that in these steps described above, as well as below, the acquisition of the k-space data may be described in terms of determining functional magnetic resonance imaging data. It is understood that during this process one or more anatomical magnetic resonance images of the subject's brain may also be acquired. For example, a magnetic resonance image which shows proton density as well as T1 or T2-weighting may be used to map the physical structure of the subject's brain. This for example may be performed by acquiring additional k-space data using additional pulse sequence commands that are configured for acquiring such an image. This may be used for developing an anatomical structure of the brain, which is then used for the mirror imaging process. The acquisition of k-space data during functional imaging may also involve acquiring k-space such that various images and curves (such as the Hemodynamic response function) can be measured. The exact measurements are dependent upon the type of functional magnetic resonance imaging protocol chosen.

In an embodiment a tonotopic mapping as used herein is a mapping of the audio cortex of the subject which maps the sensation of different tones to different locations within the audio cortex.

In an embodiment the distribution of audio frequency sensitivity within the audio cortex is in normal cortexes distributed in a mostly linear fashion. It is therefore possible to identify at least one abnormal stimulus region. The audio cortex of a subject may also exhibit symmetry. Abnormal stimulus regions may also be detected by comparing tonotopic mappings from different hemispheres of the subject's brain.

In an embodiment the baseline k-space data may be acquired before audio or sensory stimulation is provided to the subject. The baseline functional magnetic resonance image may be used as a baseline measurement for subsequent functional magnetic resonance imaging where controlled stimulation, audio stimulation, is provided to the subject.

Embodiments may be beneficial in treating tinnitus. Tinnitus, a common symptom of clinically heterogeneous pathologies, is defined as the conscious perception of an auditory sensation (experience of ringing in the ear) in the absence of a corresponding external stimulus. Tinnitus is a common problem. It affects about 15% to 20% of people and is especially common in older adults. Tinnitus negatively affects emotional health, and social well-being, and can cause psychological distress, while exerting substantial individual, and societal financial burden.

Tinnitus can arise from pathological changes along the entire auditory pathway. Tinnitus is usually caused by an underlying physical condition that damages the hair cells, such as loud sound exposure (e.g., music speaker at a concert). But hair cell damage can also be caused by age-related hearing loss or an ear injury. Tinnitus can result in abnormal neuronal activity in central auditory pathways that can then be finally perceived as tinnitus. Tinnitus can be unilateral or bilateral but can also be described to emerge within the head. The perceived sensation can be intermittent or have a pulsatile character.

Tinnitus is traditionally treated as an ear disorder. Pharmacologic interventions are not effective and entail adverse effects. Alternatively, nonpharmacologic treatments are currently used to treat tinnitus, such as acupuncture, transcutaneous electrical nerve stimulation, deep brain stimulation, cochlear stimulation therapy, and transcranial magnetic stimulation. Regular pain treatment is seldom effective for tinnitus. Cognitive behavioral therapy may be more effective in reducing the negative impact of tinnitus on quality of life when compared to other forms of treatment. However, the certainty is moderate to low.

Advances in neuroimaging methods and development of animal models have increasingly shifted the perspective of tinnitus towards a neuronal standpoint. The frequency-specific processing in the auditory cortex and beyond the auditory cortex in six tinnitus patients and six hearing loss matched controls has been investigated using task-based fMRI to perform tonotopic mapping and compared the magnitude and tuning of frequency-specific responses between the two groups. This demonstrates that tinnitus can be associated with reduced inhibition in the auditory pathway.

The inhibition problem is often referred to as a phantom problem, just like the phantom arm pain caused by an amputated arm, even although the arm is gone. The loss of the hair cells in phantom ear ringing could be compared to the loss of the arm in phantom arm pain.

In clinical management of tinnitus, some cases may be due to an inhibition problem in signal-processing between the auditive cortex and the prefrontal cortex.

Using MRI, it is possible to distinguish an anatomical ear problem from an inhibition problem in signal-processing between the auditive cortex and the prefrontal cortex. In other words, by means of MRI it is possible to distinguish phantom tinnitus from anatomical ear problem. Examples may provide a means to treat phantom tinnitus.

In another embodiment execution of the machine-executable instructions further causes the computational system to render the at least one subject-specific sensory stimulus control commands as an audio file. This for example could be rendered when the subject is within the magnetic resonance imaging system. However, this could be presented to the subject at home or during a therapy session. In the case where it is simply an audio file, the subject could replay this audio file on a computer or stereo system or through headphones and have the benefit of modifying the neural activity in the at least one abnormal stimulus region.

In other embodiments the subject-specific sensory stimulus control commands also contain video and other virtual reality images or commands. These may for example be used to augment the effectiveness of the audio file.

In another embodiment the medical system further comprises a noise cancellation system which is configured for reducing magnetic field gradient noise while controlling the sensory stimulus system with the one of the predetermined set of sensory stimulus control commands. During the acquisition of the k-space data the magnetic resonance imaging system may produce loud noises, in particular, when magnetic field gradients are produced by magnetic resonance imaging systems' gradient coils. As time-dependent currents are ramped up and down within the gradient coils, this may produce mechanical noise. The use of a noise cancellation system may help to reduce this noise and have a reduction on its effect during the functional magnetic resonance imaging.

In another embodiment each of the predetermined set of sensory stimulus control commands is configured to control the sensory stimulus system to provide a sequence of audio tones of predetermined duration, volume, timbre, and pitch. In this embodiment the predetermined set of sensory stimulus control commands contains a variety of audio tones, sounds, durations, timbres and pitches to detect which of these sounds have the biggest effect on neural activity within that at least one abnormal stimulus region. This may be beneficial because it provides a means of effectively searching for the best audio tones to treat the subject's tinnitus.

In another embodiment the medical system further comprises a subject interface that is configured for receiving a signal from the subject descriptive of the sensation of tinnitus as experienced by the subject. Execution of the machine-executable instructions further causes the computational system to receive the signal from the subject interface while control of the sensory stimulus system with one of the predetermined set of sensory stimulus control commands and also to adjust the numerical score using the signal.

In this embodiment the subject may provide a signal to indicate whether the subject has the sensation of the tinnitus or not. By providing this feedback this may be used to augment the measurements on the subject's brain for neural activity in the at least one abnormal stimulus region. So in this way, the subject's subjective experience of the tinnitus as well as using the measurements directly from the functional magnetic resonance imaging are combined and then used to select the best sensory stimulus control commands for modifying the neural activity in the at least one abnormal stimulus region.

In another embodiment execution of the machine-executable instructions are configured to cause the sensory stimulus system to repeat the one of the predetermined set of sensory stimulus control commands while the magnetic resonance imaging system is disabled. The signal is received when the magnetic resonance imaging system is disabled. A magnetic resonance imaging system may be quite noisy. In particular there may be gradient coil noise during the acquisition of the k-space data. In this embodiment the magnetic resonance imaging system is disabled and the sensory stimulus is still presented to the subject. The subject is then able to signal whether the subject senses the tinnitus or not. This provides a means of providing the subjective subject feedback without the interference of the noise of the magnetic resonance imaging system operating.

In another embodiment the medical system further comprises a physiological sensor configured for acquiring physiological data descriptive of the subject. Execution of the machine-executable instructions further causes the computational system to acquire the physiological data while controlling the sensory stimulus with the one of the predetermined set of sensory stimulus control commands. The assigning of a numerical score to the one of the predetermined set of sensory stimulus control commands further comprises increasing the score if the physiological data is outside of a predetermined value range. This embodiment may be beneficial because the physiological sensor may be used to measure an involuntary physiological response of the subject. The combination of this with the functional magnetic resonance imaging data may increase the possibility of selecting audio stimulus which is effective in reducing the subject's tinnitus.

The physiological sensor could include a sensor for measuring such things as a breathing rate, a heart rate, a skin conductivity, or an eye dilation for example.

In another embodiment the sensory stimulus system further comprises a visual stimulus system configured for providing visual stimulus to the subject. Each of the predetermined set of sensory stimulus control commands are configured to control the sensory stimulus system to present a predetermined visual stimulus to the subject. In addition to presenting a variety of audio signals to the subject, video signals and stimulus can be presented as well. This may be beneficial because it may also provide a means for associating particular sounds with a visual cue which may help the subject's brain to stimulate the neural activity in the abnormal stimulus region. For example, the majority of people have extremely sensitive relative pitch. A visual stimulus can be presented to the subject, which is modified according to the pitch.

For example, a position of a ball from left to right or according to height may be changed according to the pitch. If the subject has hair follicles which are damaged in the ear and is a cause of the tinnitus the subject may feel a sensation of a particular tone if the visual stimulus suggests that this tone is being played. For example, at various pitches a ball is raised to different locations. When it is raised to the location which corresponds to the neural activity in the abnormal stimulus region the subject may then have a feeling of a sensation of sound although the subject's ear is damaged. This may for example assist the subject physiologically by shutting off this portion of the brain and ending the tinnitus.

In another embodiment the sensory stimulus system further comprises a tactile stimulation system configured for providing tactile stimulus to the subject. Each of the predetermined set of sensory stimulus control commands are configured to control the sensory stimulus system to present a predetermined tactile stimulus to the subject. This embodiment may be beneficial because the tactile stimulus may also be associated to a particular frequency by the subject. This may assist in stimulating the abnormal stimulus region in some subjects.

In another embodiment execution of the machine-executable instructions further causes the computational system to identify the at least one abnormal stimulus region with the tonotopic mapping by detecting a deviation beyond a predetermined threshold in comparison to a tonotopic anatomical atlas. The tonotopic anatomical atlas may for example be an anatomical atlas of a brain upon which is mapped the average location of particular tones within the audio cortex. The tonotopic mapping of the subject could for example be registered to the tonotopic anatomical atlas and particular auditory responses mapped in the tonotopic mapping may be compared to the tonotopic anatomical atlas. If the two vary by more than a predetermined amount then this region may for example be identified as an abnormal stimulus region.

In another embodiment execution of the machine-executable instructions further causes the computational system to acquire initial k-space data by controlling the magnetic resonance imaging system with the pulse sequence commands. Execution of the machine-executable instructions further causes the computational system to reconstruct an initial functional magnetic resonance image from the initial k-space data. Execution of the machine-executable instructions further causes the computational system to repeatedly acquire tonotopic map k-space data by controlling the magnetic resonance imaging system with the pulse sequence commands while the sensory stimulus system is controlled with one of the tonotopic map commands.

Execution of the machine-executable instructions further causes the computational system to repeatedly control the sensory stimulus system with one of the predetermined set of tonotopic map commands during acquisition of the tonotopic map k-space data. Each of the predetermined set of tonotopic map commands are configured to control the sensory stimulus system to present a predetermined audio frequency to the subject. Execution of the machine-executable instructions further causes the computational system to repeatedly reconstruct an audio frequency-specific functional magnetic resonance image from the tonotopic map k-space data and the initial functional magnetic resonance image. The audio frequency-specific functional magnetic resonance image then provides a map of where in the auditory cortex the subject's brain is active for a particular frequency of auditory stimulus.

Execution of the machine-executable instructions further causes the computational system to construct the tonotopic map using the difference between an audio frequency-specific functional magnetic resonance image for each of the set of tonotopic map commands and the initial functional magnetic resonance image. The tonotopic map is then a mapping of which portions of the subject's brain are active when the subject receives a particular frequency of audio stimulus.

In another embodiment execution of the machine-executable instructions further causes the computational system to acquire calibration k-space data by controlling the magnetic resonance imaging system with the pulse sequence commands. Execution of the machine-executable instructions further causes the computational system to reconstruct a calibration functional magnetic resonance image from the baseline k-space data. Execution of the machine-executable instructions further causes the computational system to acquire control k-space data by controlling the magnetic resonance imaging system with the pulse sequence commands.

Execution of the machine-executable instructions further causes the computational system to control the sensory stimulus system with the subject-specific sensory stimulus control commands during the acquisition of the control k-space data. Execution of the machine-executable instructions further causes the computational system to reconstruct a control functional magnetic resonance image from the control k-space data and the calibration k-space data. The control functional magnetic resonance image is essentially a map which indicates stimulus in the audio cortex of the subject when the sensory stimulus system is controlled with the subject-specific sensory stimulus control commands. Execution of the machine-executable instructions further causes the computational system to repeat the machine-executable instructions at a time interval greater than two weeks to obtain the control functional magnetic resonance images.

These images may be combined as a function of time to form a control functional magnetic resonance image sequence. The control functional magnetic resonance image sequence may be used to illustrate how the subject is responding to the sensory stimulus over a period of time. This may for example be used to map exposure to the stimulus caused by the subject-specific sensory stimulus control commands over a period of time. This for example may be used to measure the effectiveness of the particular audio sequence being presented to the subject.

In another embodiment the memory further contains an artificial intelligence module configured to output a progress score in response to receiving the control functional magnetic resonance image sequence as an input. The progress score is descriptive of a change in neural activity in the abnormal stimulus region. Execution of the machine-executable instructions further causes the computational system to receive the progress score in response to inputting the control functional magnetic resonance image sequence into the artificial intelligence module. This may be beneficial because it may provide an effective means of monitoring the subject's progress over a period of time.

The artificial intelligence module could for example be a convolutional neural network designed for image classification with its output layer trained to assign the progress score instead of the image classification. For example, the VGG family of neural networks such as VGG-11, VGG-13, or VGG-16 would be suitable. Other suitable types of image recognition networks would be the ResNet-50 neural network. For example, instead of providing specific image classifications the outputs could be trained to output the progress score in binary. Another possibility would be to assign one classification and modify the output layer such that it outputs a continuous value. This continuous value could then also be interpreted as a progress score. There are a variety of ways to configure this in a functional way. For training data various case studies where the control functional magnetic resonance images are collected, a human operator could assign the progress score using a manual assessment in the change of neural activity in the at least one abnormal stimulus region. It could for example again be performed by comparing it to a baseline measurement and then assigning it a numerical score.

In another embodiment the magnetic resonance imaging protocol is a blood oxygenation level dependent functional magnetic resonance imaging protocol. This protocol is configured for measuring neural activity within one or more volumes selected within the excitation region of interest. The one or more volumes are a region or sub-regions of the excitation region of interest. For example, when a subject is experiencing some stimulus different portions of the subject's brain may become active. There may be advantages in measuring multiple locations and correlating their behavior to determine the neural activity metric.

In another aspect the invention provides for a method of operating the medical system. The medical system comprises a magnetic resonance imaging system that is configured for acquiring k-space data descriptive of a brain of a subject. The medical system further comprises a sensory stimulus system that is configured for providing audio stimulation to the subject.

The method comprises receiving a tonotopic mapping of an audio cortex mapping of the subject. The tonotopic mapping comprises a spatial mapping descriptive of auditory frequency-sensitive responses for the subject. The method further comprises identifying at least one abnormal stimulus region within the tonotopic mapping. The method further comprises acquiring baseline k-space data by controlling the magnetic resonance imaging system with the pulse sequence commands. The method further comprises reconstructing a baseline functional magnetic resonance image from the baseline k-space data.

The method further comprises repeatedly acquiring reference k-space data by controlling the magnetic resonance imaging system with the pulse sequence commands while the sensory stimulus system is controlled with the one of a predetermined set of sensory stimulus control commands. The pulse sequence commands are configured to control the magnetic resonance imaging system to acquire the k-space data according to a functional magnetic resonance imaging protocol for measuring brain activity in an audio cortex of the subject. The method further comprises repeatedly controlling the sensory stimulus system with one of a predetermined set of sensory stimulus control commands during acquisition of the reference k-space data. Each of the predetermined set of sensory stimulus control commands are configured to control the sensory stimulus system to present a predetermined audio stimulation to the subject.

The method further comprises repeatedly reconstructing a reference functional magnetic resonance image from the reference k-space data. The method further comprises repeatedly assigning a numerical score to the one of a predetermined set of sensory stimulus control commands by determining a change in neural activity in the at least one abnormal stimulus region between the baseline functional magnetic resonance image and the reference functional magnetic resonance image, the score being representative of a decrease or increase in the neural activity in the at least one abnormal stimulus region.

The method further comprises constructing at least one subject-specific sensory stimulus control command by selecting sensory stimulus control commands from the predetermined set of sensory stimulus control commands that maximize a change in the neural activity in the at least one abnormal stimulus region using the numerical score for each repetition of the predetermined set of sensory stimulus control commands.

In another aspect the invention provides for a computer program that comprises machine-executable instructions for execution by a computational system controlling a medical system. The computer program may for example be stored on a non-transitory storage medium. The medical system comprises a magnetic resonance imaging system that is configured for acquiring k-space data of a brain of a subject. The medical system further comprises a sensory stimulus system configured for providing audio stimulation to the subject.

Execution of the machine-executable instructions causes the computational system to receive a tonotopic mapping of an audio cortex mapping of the subject. The tonotopic mapping comprises a spatial mapping descriptive of auditory frequency-sensitive responses for the subject. Execution of the machine-executable instructions further causes the computational system to identify at least one abnormal stimulus region within the tonotopic mapping. Execution of the machine-executable instructions further causes the computational system to acquire baseline k-space data by controlling the magnetic resonance imaging system with pulse sequence commands. The pulse sequence commands are configured to control the magnetic resonance imaging system to acquire the k-space data according to a functional magnetic resonance image protocol for measuring brain activity in the audio cortex of the subject. Execution of the machine-executable instructions further causes the computational system to reconstruct a baseline functional magnetic resonance image from the baseline k-space data.

Execution of the machine-executable instructions further causes the computational system to repeatedly acquire reference k-space data by controlling the magnetic resonance imaging system with the pulse sequence commands while the sensory stimulus system is controlled with the one of a predetermined set of sensory stimulus control commands. Execution of the machine-executable instructions further causes the computational system to repeatedly control the sensory stimulus system with one of a predetermined set of sensory stimulus control commands during acquisition of the reference k-space data. Each of the predetermined set of sensory stimulus control commands are configured to control the sensory stimulus system to present a predetermined audio stimulation to the subject. Execution of the machine-executable instructions further causes the computational system to repeatedly reconstruct a reference functional magnetic resonance image from the reference k-space data. Execution of the machine-executable instructions further causes the computational system to repeatedly assign a numerical score to the one of a predetermined set of sensory stimulus control commands by detecting a change in the neural activity in the at least one abnormal stimulus region by using the baseline functional magnetic resonance image and the reference functional magnetic resonance image. The score is representative of a decrease or increase in neural activity in the at least one abnormal stimulus region.

Execution of the machine-executable instructions further causes the computational system to construct at least one subject-specific sensory stimulus control command by selecting sensory stimulus control commands from the predetermined set of sensory stimulus control commands that maximize a change in the neural activity in the at least one abnormal stimulus region by using the numerical score for each repetition of the predetermined set of sensory stimulus control commands to select it.

It is understood that one or more of the aforementioned embodiments of the invention may be combined as long as the combined embodiments are not mutually exclusive.

As will be appreciated by one skilled in the art, aspects of the present invention may be embodied as an apparatus, method or computer program product. Accordingly, aspects of the present invention may take the form of an entirely hardware embodiment, an entirely software embodiment (including firmware, resident software, micro-code, etc.) or an embodiment combining software and hardware aspects that may all generally be referred to herein as a "circuit," "module" or "system." Furthermore, aspects of the present invention may take the form of a computer program product embodied in one or more computer readable medium(s) having computer executable code embodied thereon.

Any combination of one or more computer readable medium(s) may be utilized. The computer readable medium may be a computer readable signal medium or a computer readable storage medium. A `computer-readable storage medium' as used herein encompasses any tangible storage medium which may store instructions which are executable by a processor or computational system of a computing device. The computer-readable storage medium may be referred to as a computer-readable non-transitory storage medium. The computer-readable storage medium may also be referred to as a tangible computer readable medium. In some embodiments, a computer-readable storage medium may also be able to store data which is able to be accessed by the computational system of the computing device. Examples of computer-readable storage media include, but are not limited to: a floppy disk, a magnetic hard disk drive, a solid state hard disk, flash memory, a USB thumb drive, Random Access Memory (RAM), Read Only Memory (ROM), an optical disk, a magneto-optical disk, and the register file of the computational system. Examples of optical disks include Compact Disks (CD) and Digital Versatile Disks (DVD), for example CD-ROM, CD-RW, CD-R, DVD-ROM, DVD-RW, or DVD-R disks. The term computer readable-storage medium also refers to various types of recording media capable of being accessed by the computer device via a network or communication link. For example, data may be retrieved over a modem, over the internet, or over a local area network. Computer executable code embodied on a computer readable medium may be transmitted using any appropriate medium, including but not limited to wireless, wire line, optical fiber cable, RF, etc., or any suitable combination of the foregoing.

A computer readable signal medium may include a propagated data signal with computer executable code embodied therein, for example, in baseband or as part of a carrier wave. Such a propagated signal may take any of a variety of forms, including, but not limited to, electro-magnetic, optical, or any suitable combination thereof. A computer readable signal medium may be any computer readable medium that is not a computer readable storage medium and that can communicate, propagate, or transport a program for use by or in connection with an instruction execution system, apparatus, or device.

'Computer memory' or 'memory' is an example of a computer-readable storage medium. Computer memory is any memory which is directly accessible to a computational system. `Computer storage' or 'storage' is a further example of a computer-readable storage medium. Computer storage is any non-volatile computer-readable storage medium. In some embodiments computer storage may also be computer memory or vice versa.

A `computational system' as used herein encompasses an electronic component which is able to execute a program or machine executable instruction or computer executable code. References to the computational system comprising the example of "a computational system" should be interpreted as possibly containing more than one computational system or processing core. The computational system may for instance be a multi-core processor. A computational system may also refer to a collection of computational systems within a single computer system or distributed amongst multiple computer systems. The term computational system should also be interpreted to possibly refer to a collection or network of computing devices each comprising a processor or computational systems. The machine executable code or instructions may be executed by multiple computational systems or processors that may be within the same computing device or which may even be distributed across multiple computing devices.

Machine executable instructions or computer executable code may comprise instructions or a program which causes a processor or other computational system to perform an aspect of the present invention. Computer executable code for carrying out operations for aspects of the present invention may be written in any combination of one or more programming languages, including an object-oriented programming language such as Java, Smalltalk, C++ or the like and conventional procedural programming languages, such as the "C" programming language or similar programming languages and compiled into machine executable instructions. In some instances, the computer executable code may be in the form of a high-level language or in a pre-compiled form and be used in conjunction with an interpreter which generates the machine executable instructions on the fly. In other instances, the machine executable instructions or computer executable code may be in the form of programming for programmable logic gate arrays.

The computer executable code may execute entirely on the user's computer, partly on the user's computer, as a stand-alone software package, partly on the user's computer and partly on a remote computer or entirely on the remote computer or server. In the latter scenario, the remote computer may be connected to the user's computer through any type of network, including a local area network (LAN) or a wide area network (WAN), or the connection may be made to an external computer (for example, through the Internet using an Internet Service Provider).

Aspects of the present invention are described with reference to flowchart illustrations and/or block diagrams of methods, apparatus (systems) and computer program products according to embodiments of the invention. It is understood that each block or a portion of the blocks of the flowchart, illustrations, and/or block diagrams, can be implemented by computer program instructions in form of computer executable code when applicable. It is further understood that, when not mutually exclusive, combinations of blocks in different flowcharts, illustrations, and/or block diagrams may be combined. These computer program instructions may be provided to a computational system of a general-purpose computer, special purpose computer, or other programmable data processing apparatus to produce a machine, such that the instructions, which execute via the computational system of the computer or other programmable data processing apparatus, create means for implementing the functions/acts specified in the flowchart and/or block diagram block or blocks.

These machine executable instructions or computer program instructions may also be stored in a computer readable medium that can direct a computer, other programmable data processing apparatus, or other devices to function in a particular manner, such that the instructions stored in the computer readable medium produce an article of manufacture including instructions which implement the function/act specified in the flowchart and/or block diagram block or blocks.

The machine executable instructions or computer program instructions may also be loaded onto a computer, other programmable data processing apparatus, or other devices to cause a series of operational steps to be performed on the computer, other programmable apparatus or other devices to produce a computer implemented process such that the instructions which execute on the computer or other programmable apparatus provide processes for implementing the functions/acts specified in the flowchart and/or block diagram block or blocks.

A `user interface' as used herein is an interface which allows a user or operator to interact with a computer or computer system. A `user interface' may also be referred to as a 'human interface device.' A user interface may provide information or data to the operator and/or receive information or data from the operator. A user interface may enable input from an operator to be received by the computer and may provide output to the user from the computer. In other words, the user interface may allow an operator to control or manipulate a computer and the interface may allow the computer to indicate the effects of the operator's control or manipulation. The display of data or information on a display or a graphical user interface is an example of providing information to an operator. The receiving of data through a keyboard, mouse, trackball, touchpad, pointing stick, graphics tablet, joystick, gamepad, webcam, headset, pedals, wired glove, remote control, and accelerometer are all examples of user interface components which enable the receiving of information or data from an operator.

A `hardware interface' as used herein encompasses an interface which enables the computational system of a computer system to interact with and/or control an external computing device and/or apparatus. A hardware interface may allow a computational system to send control signals or instructions to an external computing device and/or apparatus. A hardware interface may also enable a computational system to exchange data with an external computing device and/or apparatus. Examples of a hardware interface include, but are not limited to: a universal serial bus, IEEE 1394 port, parallel port, IEEE 1284 port, serial port, RS-232 port, IEEE-488 port, Bluetooth connection, Wireless local area network connection, TCP/IP connection, Ethernet connection, control voltage interface, MIDI interface, analog input interface, and digital input interface.

A 'display' or `display device' as used herein encompasses an output device or a user interface adapted for displaying images or data. A display may output visual, audio, and or tactile data. Examples of a display include, but are not limited to: a computer monitor, a television screen, a touch screen, tactile electronic display, Braille screen,

Cathode ray tube (CRT), Storage tube, Bi-stable display, Electronic paper, Vector display, Flat panel display, Vacuum fluorescent display (VF), Light-emitting diode (LED) displays, Electroluminescent display (ELD), Plasma display panels (PDP), Liquid crystal display (LCD), Organic light-emitting diode displays (OLED), a projector, and Head-mounted display.

Medical imaging data is defined herein as being recorded measurements made by a tomographic medical imaging system descriptive of a subject. The medical imaging data may be reconstructed into a medical image. A medical image id defined herein as being the reconstructed two- or three-dimensional visualization of anatomic data contained within the medical imaging data. This visualization can be performed using a computer.

K-space data is defined herein as being the recorded measurements of radio frequency signals emitted by atomic spins using the antenna of a Magnetic resonance apparatus during a magnetic resonance imaging scan. Magnetic resonance data is an example of tomographic medical image data.

A Magnetic Resonance image (MRI) or MR image is defined herein as being the reconstructed two-, three-, or four-dimensional visualization of anatomic data contained within the magnetic resonance imaging data. This visualization can be performed using a computer.

### BRIEF DESCRIPTION OF THE DRAWINGS

In the following preferred embodiments of the invention will be described, by way of example only, and with reference to the drawings in which:
Fig. 1 illustrates an example of a medical system;
Fig. 2 shows a flow chart which illustrates a method of using the medical system of Fig. 1;
Fig. 3 illustrates an example of a personal media system; and
Fig. 4 illustrates a further example of a medical system.

### DESCRIPTION OF EMBODIMENTS

Like numbered elements in these Figs are either equivalent elements or perform the same function. Elements which have been discussed previously will not necessarily be discussed in later Figs if the function is equivalent.

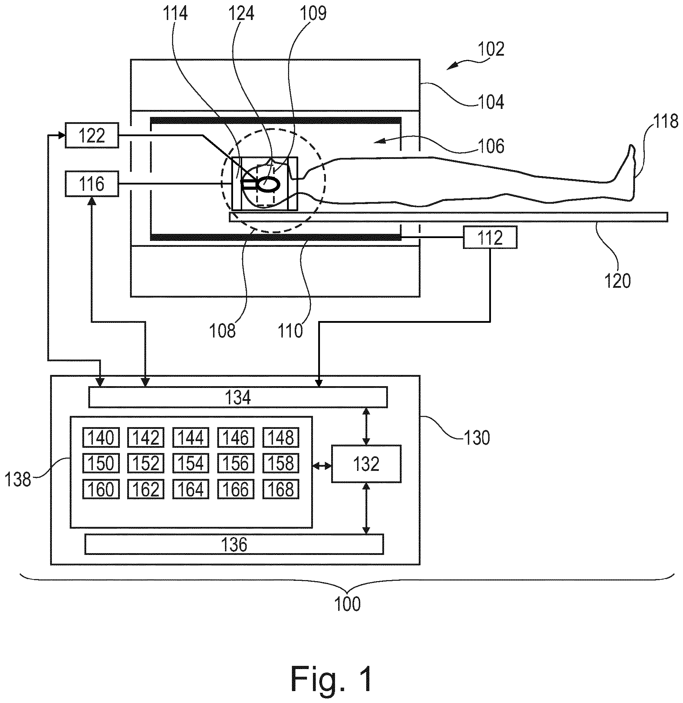

Fig. 1 illustrates an example of a medical instrument 100. The medical instrument 100 is shown as comprising a magnetic resonance imaging system 102 as well as a computer 130.

The magnetic resonance imaging system 102 comprises a magnet 104. The magnet 104 is a superconducting cylindrical type magnet with a bore 106 through it. The use of different types of magnets is also possible; for instance it is also possible to use both a split cylindrical magnet and a so called open magnet. A split cylindrical magnet is similar to a standard cylindrical magnet, except that the cryostat has been split into two sections to allow access to the iso-plane of the magnet, such magnets may for instance be used to provide for freer motion of a subject such as when moving the complementary limb 129. An open magnet has two magnet sections, one above the other with a space in-between that is large enough to receive a subject: the arrangement of the two sections area similar to that of a Helmholtz coil. Open magnets are popular, because the subject is less confined. Inside the cryostat of the cylindrical magnet there is a collection of superconducting coils.

Within the bore 106 of the cylindrical magnet 104 there is an imaging zone 108 where the magnetic field is strong and uniform enough to perform magnetic resonance imaging. A field of view 109 is shown within the imaging zone 108. The magnetic resonance data that is acquired typically acquired for the field of view 109. The field of view 109 is shown as imaging a brain volume of a subject 118 which is shown as being supported by a subject support 120.

Within the imaging zone 108 the head of the subject 118 is within a head coil 114. This enables the imaging of a field of view 109.

There is a sensory stimulus system 122 that is configured for providing stimulus to the subject 118 during the functional magnetic resonance imaging examinations. In this example there is a headphone 124 which provides audio stimulation to the subject 118. In some examples the headphone 124 may be noise cancelling to reduce the effect of gradient noise during the acquisition of k-space data. Other types of stimuli such as sound or also tactile stimulation may also be provided.

Within the bore 106 of the magnet there is also a set of magnetic field gradient coils 110 which is used for acquisition of preliminary magnetic resonance data to spatially encode magnetic spins within the imaging zone 108 of the magnet 104. The magnetic field gradient coils 110 connected to a magnetic field gradient coil power supply 112. The magnetic field gradient coils 110 are intended to be representative. Typically, magnetic field gradient coils 110 contain three separate sets of coils for spatially encoding in three orthogonal spatial directions. A magnetic field gradient power supply supplies current to the magnetic field gradient coils. The current supplied to the magnetic field gradient coils 110 is controlled as a function of time and may be ramped or pulsed.

Adjacent to the imaging zone 108 is a radio-frequency coil 114 for manipulating the orientations of magnetic spins within the imaging zone 108 and for receiving radio transmissions from spins also within the imaging zone 108. In this case the radio-frequency coil 114 is a head coil. The radio frequency antenna may contain multiple coil elements. The radio frequency antenna may also be referred to as a channel or antenna. The radio-frequency coil 114 is connected to a radio frequency transceiver 116. The radio-frequency coil 114 and radio frequency transceiver 116 may be replaced by separate transmit and receive coils and a separate transmitter and receiver. It is understood that the radio-frequency coil 114 and the radio frequency transceiver 116 are representative. The radio-frequency coil 114 is intended to also represent a dedicated transmit antenna and a dedicated receive antenna. Likewise, the transceiver 116 may also represent a separate transmitter and receivers. The radio-frequency coil 114 may also have multiple receive/transmit elements and the radio frequency transceiver 116 may have multiple receive/transmit channels.

The computer 130 could, for example, be a distributed computing system as well as a computer located remotely or via a cloud web service. The computer 130 could also be a workstation or computer used by a radiologist or other medical professional. The computer 130 could also be a control system for a magnetic resonance imaging system. The computer 130 is shown as comprising a computational system 132 that is intended to represent one or more computational systems that may be located in one or more locations. The computational system 132 is connected to a hardware interface 134 and an optional user interface 136. The hardware interface 134 enables the computational system 132 to control other components of the medical instrument 100 such as the magnetic resonance imaging system 102. The user interface 108 may enable an operator to control and operate and interact with the medical instrument 100.

The computational system 132 is further shown as being in communication with a memory 138. The memory 138 is intended to represent various types of memory which may be able to communicate with the computational system 132. The memory 138 is shown as containing machine-executable instructions 140. The machine-executable instructions 130 enable the computational system 132 to perform various tasks such as controlling other components of the medical instrument 100 as well as performing numerical and image processing tasks.

The memory 138 is shown as containing optional scout scan pulse sequence commands 142. This could be used to acquire scout scan k-space data, which is then reconstructed into a scout image 144. The scout image 144 may not necessarily necessary in all examples. However, the scout image 144 could be used for correctly identifying the location of the brain and could also be used during the portions of when the various functional regions of the brain are determined. For example, it may provide a proton density image which provides a very good structural image of how the brain is structured, which may be useful in using the mirror imaging technique to transfer one functional region from one hemisphere of the brain to the other.

The memory 138 is further shown as containing pulse sequence commands 146 that are according to a functional magnetic resonance imaging protocol that is used for measuring the brain activity of the subject 118. The pulse sequence commands 146 are used throughout and it is understood that the various minor parameters, such as adjustments of the field of view 109 and other properties, can take place.

The pulse sequence commands are configured or adjusted to acquire k-space data according to a functional magnetic resonance imaging protocol for measuring brain activity in the audio cortex of the subject. The memory 138 is further shown as containing a tonotopic mapping 148 for the subject 118. The tonotopic mapping identifies specific regions of the brain which respond to particular auditory frequencies. The memory 138 is further shown as containing an abnormal stimulus region 150 that is identified in the tonotopic mapping 148. This for example could be a region which does not respond as expected to a particular audio frequency or it may refer to a region which is shifted from the norm when comparing the results from many other subjects.

The memory 138 is further shown as containing baseline k-space data 152 and a baseline functional magnetic resonance image 154. The baseline functional magnetic resonance image 154 is a functional magnetic resonance image when the subject is not presented with auditory stimulus from the sensory stimulus system 122. The memory 138 is further shown as containing reference k-space data 156 and a corresponding reference functional magnetic resonance image 158 that has been prepared for one 162 of a predetermined set of sensory stimulus commands 160. The reference k-space data 156 and the reference functional magnetic resonance image 158 may be acquired for a number of different sensory stimulus commands.

The predetermined set of sensory stimulus commands 160 and the one 162 of the predetermined set of sensory stimulus commands are also shown as being stored in the memory 138. The memory 138 is further shown as containing a numerical score 164 for each of the predetermined set of sensory stimulus commands 160. The memory 138 is further shown as containing a numerical score 164 for each of the predetermined set of sensory stimulus commands. The numerical score 164 may be based on an increase or decrease in the neural activity in the abnormal stimulus region 150. The memory 138 is further shown as containing at least one subject-specific sensory stimulus control command 166 that has been constructed by using the numerical score 164 to select the predetermined set of sensory stimulus commands 160 that result in the largest increase or decrease in the neural activity in the abnormal stimulus region 150. The memory 138 is shown as further containing an optional audio file 168 that was rendered from the at least one subject-specific sensory stimulus control commands 166.

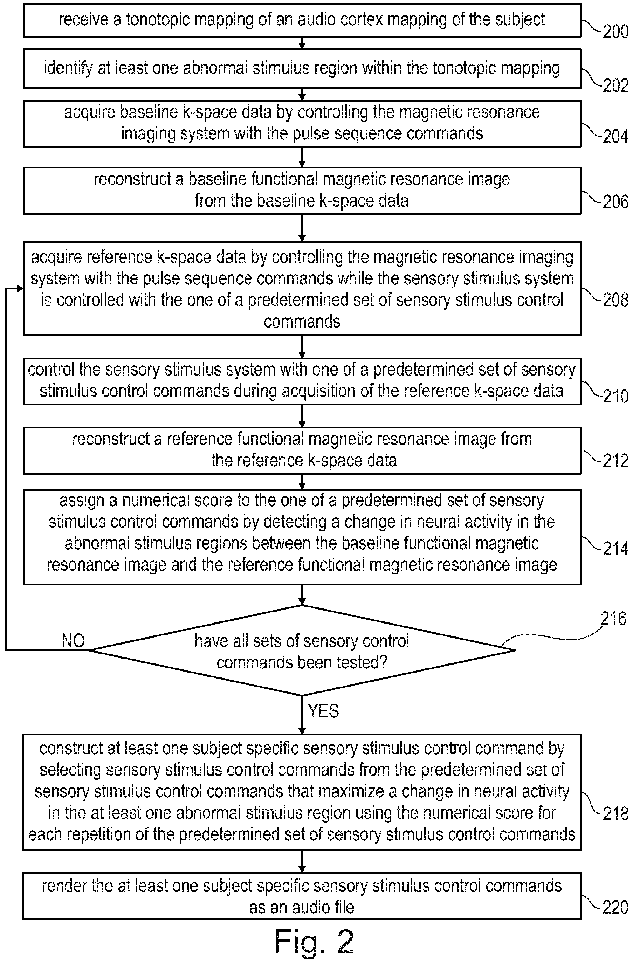

Fig. 2 shows a flowchart which illustrates a method of operating the medical system of Fig. 1. First, in step 200, the tonotopic mapping 148 is received. The tonotopic mapping comprises a spatial mapping that is descriptive of auditory frequency sensitivity responses for the subject. Next, in step 202, at least one abnormal stimulus region 150 is identified within the tonotopic mapping. Then, in step 204, the baseline k-space data 152 is acquired by controlling the magnetic resonance imaging system with the pulse sequence commands 146. Then, in step 206, the baseline functional magnetic resonance image 154 is reconstructed from the baseline k-space data 152. The method then proceeds to step 208. In step 208 the reference k-space data is acquired by controlling the magnetic resonance imaging system 102 with the pulse sequence commands 146.

Next, in step 210, the sensory stimulus system 122 is controlled with one 162 of the predetermined set of sensory stimulus control commands 160 while the reference k-space data 156 is acquired. Next, in step 212, the reference functional magnetic resonance image 158 is reconstructed from the reference k-space data 156. Next, in step 214, the numerical score 164 is assigned to the one 162 of the predetermined set of sensory stimulus commands 160 by detecting a change in the neural activity in the abnormal stimulus region 150 by comparing the reference functional magnetic resonance image 158 with the baseline functional magnetic resonance image 154.

Next the method proceeds to decision box 216 and the question is: `have all of the sensory stimulus control commands been tested?' If the answer is 'no' the method proceeds back to step 208. If the answer is 'yes' the method proceeds to step 218. In step 218 the method further comprises constructing 218 at least one subject-specific sensory stimulus control command 166 by selecting the sensory stimulus control commands from the predetermined set of sensory stimulus control commands 160 that maximize a change in neural activity in the at least one abnormal stimulus region 150. The method may then proceed to optional step 220. In step 220 an audio file 168 is optionally rendered from the at least one subject-specific sensory stimulus control commands 166.

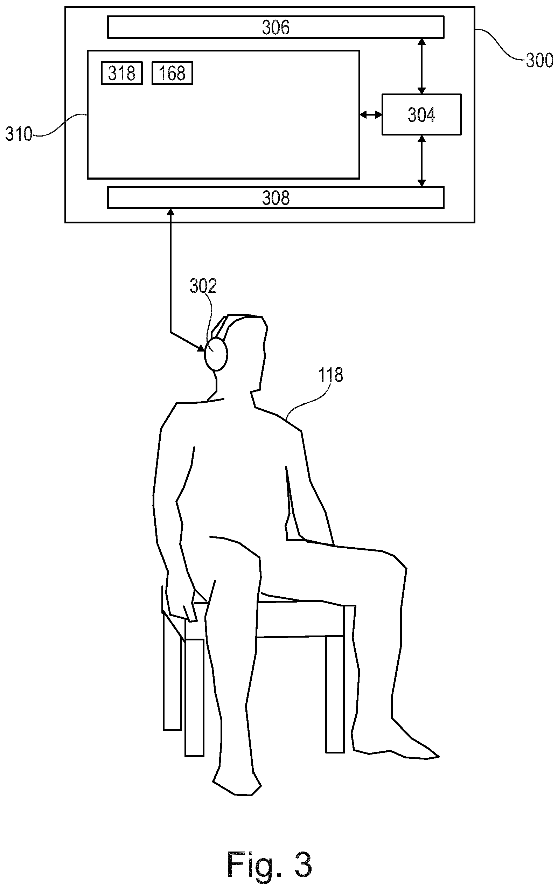

Fig. 3 illustrates an example of a personal media system 300. The personal media system 300 is shown as being connected to headphones 302 being worn by the subject 118. The personal media system 300 is shown as further comprising a processor 304 that is in connection with a user interface 306 and an audio interface 308. There is additionally a memory 310 which is storing local machine-executable instructions 312 and the audio file 168. The local machine-executable instructions 312 enable the processor 304 to control the personal media system 300 as well as to play the audio file 168 using the audio interface 308. The medical system 100 in Fig. 1 may be used to construct the audio file 168. Using the functional magnetic resonance imaging the audio file 168 has been rendered such that it optionally stimulates the normal stimulus region 150. This may have the effect that the subject 118 can play the audio file 168 repeatedly and this may have the effect of reducing or eliminating the tinnitus.

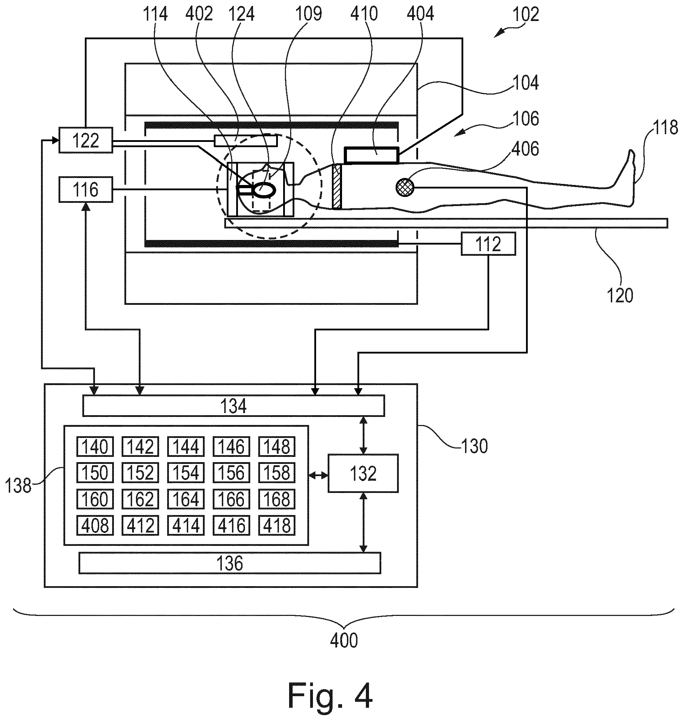

Fig. 4 illustrates a further example of a medical system 400. The medical system 400 depicted in Fig. 4 is similar to the medical system 100 depicted in Fig. 1 except that it comprises additional components. In Fig. 4 there is additionally a visual stimulus system (i.e., a display) 402 and a tactile stimulus system 404 that are connected to the sensory stimulus system 122. In this example the sensory stimulus system 122 is able to provide audio, visual, and tactile stimulation to the subject 118 instead of just auditory stimulation. There is also additionally a squeeze ball 406 which functions as a subject interface. The subject interface 406 can be used by the subject 118 to indicate if the subject has the sensation of tinnitus or not. Additionally, there is a physiological sensor 410 which measures physiological data 412. During the measurement of the k-space data for functional magnetic resonance imaging involuntary physiological responses of the subject 118 can be measured and considered also.

The memory 138 is further shown as containing the physiological data 412 as well as a signal 408 from the squeeze ball 406. The memory 138 is further shown as containing an artificial intelligence module 416 that for instance outputs a progress score 416 for the subject 118 in response to receiving as input control functional magnetic resonance image sequence 418. For example, after the at least one subject-specific sensory stimulus control commands 166 have been determined the subject 118 can be treated, as was illustrated in Fig. 3, and then at periodic intervals the response of the subject's abnormal stimulus regions 115 can be measured. This can be used to construct the control functional magnetic resonance image sequence 418. This may be a good measure if there is a need to modify the subject-specific sensory stimulus control commands 166 or not.

While the invention has been illustrated and described in detail in the drawings and foregoing description, such illustration and description are to be considered illustrative or exemplary and not restrictive; the invention is not limited to the disclosed embodiments.

Other variations to the disclosed embodiments can be understood and effected by those skilled in the art in practicing the claimed invention, from a study of the drawings, the disclosure, and the appended claims. In the claims, the word "comprising" does not exclude other elements or steps, and the indefinite article "a" or "an" does not exclude a plurality. A single processor or other unit may fulfill the functions of several items recited in the claims. The mere fact that certain measures are recited in mutually different dependent claims does not indicate that a combination of these measured cannot be used to advantage. A computer program may be stored/distributed on a suitable medium, such as an optical storage medium or a solid-state medium supplied together with or as part of other hardware, but may also be distributed in other forms, such as via the Internet or other wired or wireless telecommunication systems. Any reference signs in the claims should not be construed as limiting the scope.

### REFERENCE SIGNS LIST

- 100: medical instrument
- 102: magnetic resonance imaging system
- 104: magnet
- 106: bore of magnet
- 108: imaging zone
- 109: field of view
- 110: magnetic field gradient coils
- 112: magnetic field gradient coil power supply
- 114: head coil
- 116: transceiver
- 118: subject
- 120: subject support
- 122: sensory stimulus system
- 124: headphones
- 130: computer
- 132: computational system
- 134: hardware interface
- 136: user interface
- 138: memory
- 140: machine executable instructions
- 142: scout scan pulse sequence commands
- 144: scout image
- 146: pulse sequence commands
- 148: tonotopic mapping
- 150: abnormal stimulus region identified in tonotopic mapping
- 152: baseline k-space data
- 154: baseline functional magnetic resonance image
- 156: reference k-space data
- 158: reference functional magnetic resonance image
- 160: set of predetermine sensory stimulus commands
- 162: one of the set of predetermined sensory stimulus commands
- 164: numerical scores for set of predetermined sensory stimulus commands
- 166: at least one subject specific sensory stimulus control commands
- 168: audio file
- 200: receive a tonotopic mapping of an audio cortex mapping of the subject
- 202: identify at least one abnormal stimulus region within the tonotopic mapping
- 204: acquire baseline k-space data by controlling the magnetic resonance imaging system with the pulse sequence commands
- 206: reconstruct a baseline functional magnetic resonance image from the baseline k-space data
- 208: acquire reference k-space data by controlling the magnetic resonance imaging system with the pulse sequence commands while the sensory stimulus system is controlled with the one of a predetermined set of sensory stimulus control commands
- 210: control the sensory stimulus system with one of a predetermined set of sensory stimulus control commands during acquisition of the reference k-space data
- 212: reconstruct a reference functional magnetic resonance image from the reference k-space data
- 214: assigning a numerical score to the one of a predetermined set of sensory stimulus control commands by detecting a change in neural activity in the at least one abnormal stimulus region between the baseline functional magnetic resonance image and the reference functional magnetic resonance image
- 218: construct at least one subject specific sensory stimulus control command by selecting sensory stimulus control commands from the predetermined set of sensory stimulus control commands that maximize a change in neural activity in the at least one abnormal stimulus region using the numerical score for each repetition of the predetermined set of sensory stimulus control commands
- 220: render the at least one subject specific sensory stimulus control commands as an audio file
- 300: personal media system
- 302: head phones
- 304: processor
- 306: user interface
- 308: audio interface
- 310: memory
- 312: local machine executable instructions
- 400: medical system
- 402: visual stimulus system
- 404: tactile stimulus system
- 406: squeeze ball (subject interface)
- 408: signal
- 410: physiological sensor
- 412: physiological data
- 414: artificial intelligence module
- 416: progress score
- 418: control functional magnetic resonance image sequence

## Claims

1. A medical system (100, 400) comprising:
- a magnetic resonance imaging system (102) configured for acquiring k-space data (152, 156) of a brain of a subject (118);
- a sensory stimulus system (122) configured for providing audio stimulation to the subject;
- a memory (138) storing machine executable instructions (140) and pulse sequence commands (146), wherein the pulse sequence commands are configured to control the magnetic resonance imaging system to acquire the k-space data according to a functional magnetic resonance imaging protocol for measuring brain activity in an audio cortex of the subject;
- a computational system (132) configured for controlling the medical system, wherein execution of the machine executable instructions causes the computational system to:
- receive (200) a tonotopic mapping (148) of an audio cortex mapping of the subject, wherein the tonotopic mapping comprises a spatial mapping descriptive of auditory frequency sensitive responses for the subject;
- identify (202) at least one abnormal stimulus region (150) within the tonotopic mapping;
- acquire (204) baseline k-space data (152) by controlling the magnetic resonance imaging system with the pulse sequence commands;
- reconstruct (206) a baseline functional magnetic resonance image (154) from the baseline k-space data;
wherein execution of the machine executable instructions causes the computational system to repeatedly:
- acquire (208) reference k-space data (156) by controlling the magnetic resonance imaging system with the pulse sequence commands;
- control (210) the sensory stimulus system with one (162) of a predetermined set of sensory stimulus control commands (160) during acquisition of the reference k-space data, wherein each of the predetermined set of sensory stimulus control commands are configured to control the sensory stimulus system to present a predetermined audio stimulation to the subject;
- reconstruct (212) a reference functional magnetic resonance image (158) from the reference k-space data; and
- assign (214) a numerical score (164) to the one of the predetermined set of sensory stimulus control commands by detecting a change in neural activity in the at least one abnormal stimulus region between the baseline functional magnetic resonance image and the reference functional magnetic resonance image, the score being representative of a decrease or an increase in neural activity in the at least one abnormal stimulus region; and
wherein execution of the machine executable instructions further causes the computational system to construct (218) at least one subject specific sensory stimulus control command (166) by selecting sensory stimulus control commands from the predetermined set of sensory stimulus control commands that maximize a change in neural activity in the at least one abnormal stimulus region using the numerical score for each repetition of the predetermined set of sensory stimulus control commands.

2. The medical system of any one of the preceding claims, wherein execution of the machine executable instructions further causes the computational system to render (220) the at least one subject specific sensory stimulus control commands as an audio file (168).

3. The medical system of any one of the preceding claims, wherein the medical system further comprises a noise cancellation system configured for reducing magnetic field gradient noise while controlling the sensory stimulus system with the one of the predetermined set of sensory stimulus control commands.

4. The medical system of any one of the preceding claims, wherein the each one of the predetermined set of sensory stimulus control commands is configured to control the sensory stimulus system configured to provide a sequence of audio tones of predetermined duration, volume, timber, and pitch.

5. The medical system of any one of the preceding claims, wherein the medical system further comprises a subject interface (406) configured to receive a signal (408) from the subject descriptive of the sensation of tinnitus experienced by the subject, wherein execution of the machine executable instructions further causes the computational system to:
- receive the signal from the subject interface while control the sensory stimulus system with one of a predetermined set of sensory stimulus control commands; and
- adjust the numerical score using the signal.

6. The medical system of claim 5, wherein execution of the machine executable instructions are configured to cause the sensory stimulus system to repeat the one of the predetermined set of sensor stimulus commands while the magnetic resonance imaging system is disabled, wherein the signal is received when the magnetic resonance imaging system is disabled.

7. The medical system of any one of the preceding claims, wherein the medical system further comprises a physiological sensor (410) configured for acquiring physiological data (412) descriptive of the subject, wherein execution of the machine executable instructions further causes the computational system to acquire the physiological data while controlling the sensory stimulus system with the one of the predetermined set of the sensory stimulus control commands, wherein the assigning the numerical score to the one of the predetermined set of sensory stimulus control commands further comprises increasing the score if the physiological data is outside of a predetermined value range.

8. The medical system of any one of the preceding claims, wherein the sensory stimulus system further comprises a visual stimulus system (402) configured for providing visual stimulus to the subject, wherein each of the predetermined set of sensory stimulus control commands are configured to control the sensory stimulus system to present a predetermined visual stimulus to the subject.

9. The medical system of any one of the preceding claims, wherein the sensory stimulus system further comprises a tactile stimulus system (404) configured for providing tactile stimulus to the subject, wherein each of the predetermined set of sensory stimulus control commands are configured to control the sensory stimulus system to present a predetermined tactile stimulus to the subject.

10. The medical system of any one of the preceding claims, wherein execution of the machine executable instructions further causes the computational system to identify the at least one abnormal stimulus region within the tonotopic mapping by detecting a deviation beyond a predetermined threshold in comparison to a tonotopic anatomical atlas.

11. The medical system of any one of the preceding claims, wherein execution of the machine executable instructions further causes the computational system to:
- acquire initial k-space data by controlling the magnetic resonance imaging system with the pulse sequence commands;
- reconstruct an initial functional magnetic resonance image from the initial k-space data; wherein execution of the machine executable instructions causes the computational system to repeatedly:
- acquire tonotopic map k-space data by controlling the magnetic resonance imaging system with the pulse sequence commands while the sensory stimulus system is controlled with the one of a predetermined set of tonotopic map commands;
- control the sensory stimulus system with one of the predetermined set of tonotopic map commands during acquisition of the tonotopic map k-space data, wherein each of the predetermined set of tonotopic map commands are configured to control the sensory stimulus system to present a predetermined audio frequency to the subject; and
- reconstruct an audio frequency specific functional magnetic resonance image from the tonotopic map k-space data;
wherein execution of the machine executable instructions further causes the computational system to construct the tonotopic map using a difference between the audio frequency specific functional magnetic resonance image for each of the set of tonotopic map commands and the initial functional magnetic resonance image.

12. The medical system of any one of the preceding claims, wherein execution of the machine executable instructions further causes the computational system to:
- acquire calibration k-space data by controlling the magnetic resonance imaging system with the pulse sequence commands;
- reconstruct a calibration functional magnetic resonance image from the baseline k-space data;
- acquire control k-space data by controlling the magnetic resonance imaging system with the pulse sequence commands;
control the sensory stimulus system the subject specific sensory stimulus control commands during acquisition of the control k-space data; and
- reconstruct a control functional magnetic resonance image from the control k-space data and the calibration k-space data;
- repeat the machine executable instructions at time interval greater than two weeks to obtain the control functional magnetic resonance image as a function of time to form a control functional magnetic resonance image sequence.

13. The medical system of claim 12, wherein the memory further contains an artificial intelligence module (414) configured to output a progress score (416) in response to receiving the control functional magnetic resonance image sequence as input, wherein the progress score is descriptive of a change in neural activity in the abnormal stimulus region, wherein execution of the machine executable instructions further causes the computational system to receive the progress score in response to inputting the control functional magnetic resonance image sequence into the artificial intelligence module.

14. A method of operating a medical system (100, 400), wherein the medical system comprises a magnetic resonance imaging system (102) configured for acquiring k-space data (152, 156) of a brain of a subject (118), wherein the medical system further comprises a sensory stimulus system configured for providing audio stimulation to the subject,
wherein the method comprises:
- receiving (200) a tonotopic mapping (148) of an audio cortex mapping of the subject,
wherein the tonotopic mapping comprises a spatial mapping descriptive of auditory frequency sensitive responses for the subject;
- identifying (202) at least one abnormal stimulus region (!50) within the tonotopic mapping;
- acquiring (204) baseline k-space data (152) by controlling the magnetic resonance imaging system with the pulse sequence commands;
- reconstructing (206) a baseline functional magnetic resonance image (154) from the baseline k-space data;
wherein the method further comprises repeatedly:
- acquiring (208) reference k-space data (156) by controlling the magnetic resonance imaging system with pulse sequence commands (146), wherein the pulse sequence commands are configured to control the magnetic resonance imaging system to acquire the k-space data according to a functional magnetic resonance imaging protocol for measuring brain activity in an audio cortex of the subject;
- controlling (210) the sensory stimulus system with one (162) of a predetermined set of sensory stimulus control commands (160) during acquisition of the reference k-space data, wherein each of the predetermined set of sensory stimulus control commands are configured to control the sensory stimulus system to present a predetermined audio stimulation to the subject;
- reconstructing (212) a reference functional magnetic resonance image (158) from the reference k-space data; and
- assigning (214) a numerical score (164) to the one of a predetermined set of sensory stimulus control commands by detecting a change in neural activity in the at least one abnormal stimulus region between the baseline functional magnetic resonance image and the reference functional magnetic resonance image, the score being representative of a decrease or an increase in neural activity in the at least one abnormal stimulus region; and
wherein the method further comprises constructing (218) at least one subject specific sensory stimulus control command (166) by selecting sensory stimulus control commands from the predetermined set of sensory stimulus control commands that maximize a change in neural activity in the at least one abnormal stimulus region using the numerical score for each repetition of the predetermined set of sensory stimulus control commands.

15. A computer program comprising machine executable instructions for execution by a computational system controlling a medical system (100, 400), wherein the medical system comprises a magnetic resonance imaging system (102) configured for acquiring k-space data (152, 156) of a brain of a subject (118), wherein the medical system further comprises a sensory stimulus system (122) configured for providing audio stimulation to the subject;
wherein execution of the machine executable instructions causes the computational system to:
- receive (200) a tonotopic mapping (148) of an audio cortex mapping of the subject, wherein the tonotopic mapping comprises a spatial mapping descriptive of auditory frequency sensitive responses for the subject;
- identify (202) at least one abnormal stimulus region (150) within the tonotopic mapping;
- acquire (204) baseline k-space data (152) by controlling the magnetic resonance imaging system with pulse sequence commands, wherein the pulse sequence commands are configured to control the magnetic resonance imaging system to acquire the k-space data according to a functional magnetic resonance imaging protocol for measuring brain activity in an audio cortex of the subject;
- reconstruct (206) a baseline functional magnetic resonance image (154) from the baseline k-space data;
wherein execution of the machine executable instructions causes the computational system to repeatedly:
- acquire (208) reference k-space data (156) by controlling the magnetic resonance imaging system with the pulse sequence commands;
- control (210) the sensory stimulus system with one (162) of a predetermined set of sensory stimulus control commands (160) during acquisition of the reference k-space data, wherein each of the predetermined set of sensory stimulus control commands are configured to control the sensory stimulus system to present a predetermined audio stimulation to the subject;
- reconstruct (212) a reference functional magnetic resonance image (158) from the reference k-space data; and
- assign (214) a numerical score (164) to the one of a predetermined set of sensory stimulus control commands by detecting a change in neural activity in the at least one abnormal stimulus region between the baseline functional magnetic resonance image and the reference functional magnetic resonance image, the score being representative of a decrease or an increase in neural activity in the at least one abnormal stimulus region; and
wherein execution of the machine executable instructions further causes the computational system to construct (218) at least one subject specific sensory stimulus control command (166) by selecting sensory stimulus control commands from the predetermined set of sensory stimulus control commands that maximize a change in neural activity in the at least one abnormal stimulus region using the numerical score for each repetition of the predetermined set of sensory stimulus control commands.
